# EUROPÄISCHE PATENTSCHRIFT

(11) **EP 0 797 406 B1**
(45) Veröffentlichungstag und Bekanntmachung des Hinweises auf die Patenterteilung: **02.06.1999**
(21) Anmeldenummer: 95941578.7
(22) Anmeldetag: 11.12.1995
(51) Int. Cl.: A61B 17/08

(54) **HAUT-DISTRAKTOR**
SKIN PINCHING DEVICE
DISPOSITIF PERMETTANT DE RESSERRER LA PEAU

(30) Priorität: 12.12.1994 DE 4444130
(43) Veröffentlichungstag der Anmeldung: 01.10.1997
(73) Patentinhaber: Fleischmann, Wim, 89182 Bernstadt (DE)
(72) Erfinder: Fleischmann, Wim, 89182 Bernstadt (DE)
(74) Vertreter: Mussgnug, Bernd, Dr.rer.nat.
(86) Internationale Anmeldenummer: DE9501766
(87) Internationale Veröffentlichungsnummer: WO9618345

(56) Entgegenhaltungen:
- EP-A- 0 354 599
- EP-A- 0 444 776
- WO-A-94/26173
- WO-A-95/28886
- DE-U-29 500 369
- FR-A- 2 599 238
- US-A- 2 693 795
- US-A- 3 068 869
- US-A- 3 643 655
- US-A- 3 825 010
- US-A- 4 899 761
- US-A- 5 020 933

## Beschreibung

Die Erfindung betrifft einen Haut-Distraktor gemäß dem Oberbegriff des Anspruchs 1.

Haut-Distraktoren dieser Gattung dienen dazu, bei großflächigen Wunden die Haut der Wundränder zusammenzuziehen. Das Zusammenziehen der Wundränder kann innerhalb einer kurzen Zeit von etwa 15 bis 30 Minuten erfolgen, um die Wunde vernähen zu können (US-PS 4 896 680). Dabei wird die Haut im Bereich der Wundränder unter Ausnutzung ihrer viskoelastischen Eigenschaften gedehnt. Werden die Wundränder dagegen langsam über einen Zeitraum von einigen Tagen oder Wochen zusammengezogen, so kann die Wunde unter Gewebsneubildung der Haut verschlossen werden, ohne daß die Haut bis zur viskoelastischen Dehnung beansprucht wird.

Aus der WO-A-94 26173 (Fig. 39 - 42) ist ein Haut-Distraktor der eingangs genannten Gattung bekannt. Dieser Distraktor weist zwei Backen auf, an denen jeweils lösbar ein Teil mit zwei Haken angebracht ist. Die zwei Haken werden in die Haut eingestochen und durch eine in die Haut einsteckbare Quernadel in der Haut verankert. Die Backen sitzen jeweils auf einer Gewindespindel, mittels welcher sie in ihrem gegenseitigen Abstand verstellbar sind, um die Wundränder, in welchen die Haken verankert sind, gegeneinander zu ziehen.

Bei diesem bekannten Haut-Distraktor sind die Backen zwangsläufig rechtwinklig zur Verstellrichtung angeordnet und geführt. Die Haken und die die Haken in der Haut verankernden Nadeln müssen daher exakt senkrecht zur Verstellrichtung in die Haut eingesetzt werden, um umgleichmäßige Zugspannungen in der Haut zu vermeiden. Eine Anpassung der Verankerung des Distraktors in der Haut an den Verlauf der Wundränder ist nicht möglich. Der Distraktor ist außerdem ein aufwendiges Instrument, das schwierig zu reinigen und zu sterilisieren ist.

Der Erfindung liegt die Aufgabe zugrunde, einen Haut-Distraktor so auszubilden, daß entlang der Wundränder auch eine größere Anzahl von Haken problemlos nebeneinander in die Haut eingestochen werden können.

Diese Aufgabe wird erfindungsgemäß gelöst durch die in dem Patentanspruch 1 angegebenen Merkmale.

Vorteilhafte Ausbildungen der Erfindung sind in den Unteransprüchen angegeben.

Bei dem erfindungsgemäßen Haut-Distraktor sind die Haken zur Verankerung der Backen in der Haut an einem Teil angebracht, das auswechselbar an der Backe angeordnet ist. Die Haken, die in die Haut des Patienten eingestochen werden und die eine hohe Sterilität erfordern, können auf diese Weise als Einmal-Teile hergestellt werden, die jeweils nach der Benutzung weggeworfen werden. Der übrige aufwendige Distraktor kann gereinigt, desinfiziert und mehrfach verwendet werden. Vorzugsweise werden die Haken in ein Kunststoffteil eingespritzt, so daß das nur einmal benutzbare Teil äußerst kostengünstig hergestellt werden kann. Das Auswechseln und Wegwerfen der Teile mit den Haken nach der Verwendung hat weiter den Vorteil, daß die Gefahr einer Verletzung und Infektion des Personals durch die extrem scharfen und spitzen Haken bei der Reinigung, Desinfektion und Lagerung des Distraktors ausgeschlossen ist.

Die Anordnung der Haken in gesonderten, an den Backen des Distraktors angeordneten Teilen hat weiter den Vorteil, daß die Haken von dem Distraktor getrennt in die Haut des Patienten eingestochen werden können. Das wegen der Nachgiebigkeit der Haut schwierige Einstechen der Haken wird dadurch wesentlich erleichtert. Nach dem Einstechen der Haken werden die Teile mit den Haken an den Backen befestigt. Die Befestigung kann beispielsweise mittels einer Schwalbenschwanzführung erfolgen. Andere Befestigungsmöglichkeien stehen dem Fachmann zur Verfügung.

Es sind jeweils nur ein einziger Haken, zwei Haken oder einige wenige Haken in ein gemeinsames Kunststoffteil gespritzt. Daduch wird das Einstechen der Haken in die Haut wesentlich erleichtert, da ein oder zwei Haken in jedem Fall problemlos gleichzeitig und gemeinsam nebeneinander in die Haut eingestochen werden können. Nach dem Einstechen werden die die Haken haltenden einzelnen Teile nacheinander modulartig aneinander anschließend an den Backen befestigt, z.B. in eine Schwalbenschwanz-Führung eingeschoben, um die Reihe von Haken zu bilden. Auf diese Weise kann auch eine große Zahl von Haken problemlos in einer Reihe an einer Backe angebracht werden, ohne daß das Einstechen der Haken in die Wundränder Schwierigkeiten macht. Die Breite der einzelnen jeweils einen Haken aufnehmenden Teile legt dabei den Abstand der Haken in der Reihe fest.

Die Backen können auf zwei in Verstellrichtung verlaufende parallelen Führungsstangen mittels Führungsbuchsen geführt sein, wobei die Führungsbuchsen um eine vertikale, zu der Ebene der Verstellbewegung senkrechte Achse drehbar in den Backen gelagert sind. Die Führungsstangen können dabei in den Führungsbuchsen gleitend geführt sein, wobei die Stellmittel zur Abstandverstellung gesondert vorgesehen sind. Es können auch eine oder beide Führungsstangen als Gewindespindel ausgebildet sein, wobei die entsprechenden Führungsbuchsen als Gewindebuchsen ausgebildet sind. Die als Gewindespindel ausgebildete Führungsstange dient dabei gleichzeitig als Stellmittel zur Abstandsverstellung.

Weiter können die zwei Backen an zwei Armen angeordnet sein, die in der Ebene der Verstellrichtung gegeneinander verschwenkbar miteinander verbunden sind. Dadurch ergibt sich eine besonders einfache Gestaltung des Distraktors, die die Herstellungskosten reduziert und die Handhabung vereinfacht.

Besonders einfach ist diese Ausführung, wenn die Backen in der Verstellebene unverdrehbar an den Armen befestigt sind. Die gegenseitige Verstellung der Backen erfolgt dadurch zwar auf einer Kreisbogenbahn, dies ist jedoch dann unerheblich, wenn der Verstellweg, d.h. die Breite der Wunde nicht zu groß ist und wenn die Länge der Arme, d.h. der Radius des Kreisbogens nicht zu klein ist.

Um eine Gewebsneubildung bei der Distraktion der Haut zu ermöglichen, werden die Backen des Distraktors nur langsam in kleinen Schritten mit großem zeitlichem Abstand gegeneinander bewegt. Dabei kann eine Sonde verwendet werden, die den Zustand der Haut in dem Dehnungsbereich auf der von der Wunde abgewandten Seite der Backen ermittelt. Dies kann beispielsweise eine die Hautdurchblutung oder den Sauerstoffpartialdruck bestimmende Sonde sein. Die Stellmittel zur Verstellung des Backenabstandes werden entsprechend den von der Sonde ermittelten Werten betätigt. Dadurch wird einerseits vermieden, daß die Haut zu stark gedehnt und dadurch geschädigt wird, andererseits kann das Nachstellen des Backenabstandes zum frühestmöglichen Zeitpunkt erfolgen, so daß ein optimaler Gewebsgewinn und damit eine möglichst kurze Heilungszeit erzielt werden.

Werden die Stellmittel motorisch angetrieben, so ist ein automatisches Nachstellen entsprechend den mit der Sonde ermittelten Werten möglich.

Im folgenden wird die Erfindung anhand von in der Zeichnung dargestellten Ausführungbeispielen näher erläutert. Es zeigen:
- Figur 1: eine Seitenansicht eines Haut-Distraktors,
- Figur 2: eine Draufsicht auf den Haut-Distraktor der Figur 1,
- Figur 3: eine Ansicht gemäß der Schnittlinie III-III in Figur 2,
- Figur 4: eine Draufsicht auf eine zweite Ausführung des Haut-Distraktors,
- Figur 5: einen Schnitt gemäß der Linie V-V in Figur 4,
- Figur 6: eine Draufsicht auf eine weitere Ausführung des Haut-Distraktors und
- Figur 7: schematisch eine Steuerung zum automatischen Nachstellen des Haut-Distraktors.

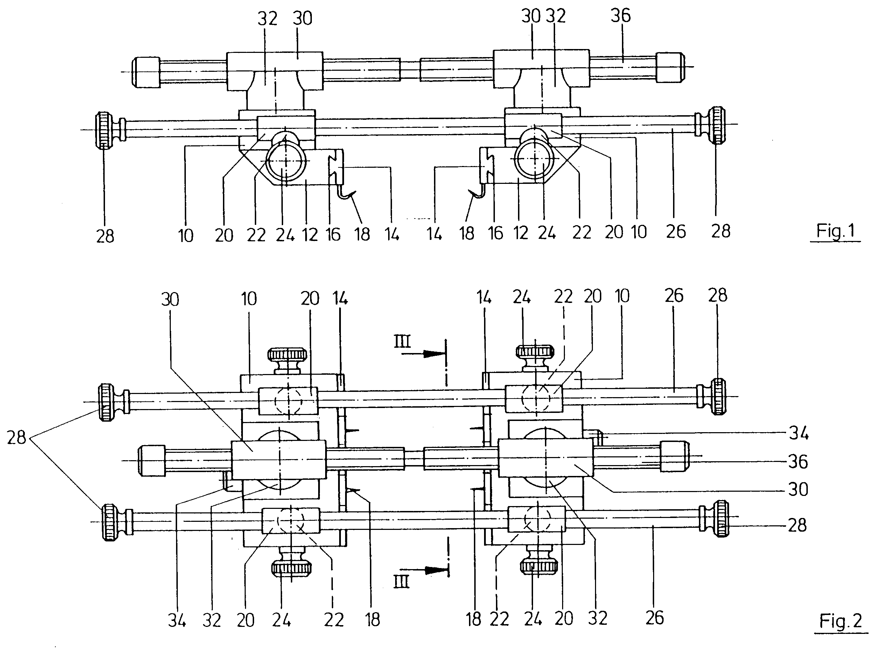

In den Figuren 1 bis 3 ist ein erstes Ausführungsbeispiel des Haut-Distraktors gezeigt.

Der Distraktor weist zwei spiegelsymmetrisch ausgebildete Backen 10 auf. Die Backen 10 sind an ihrer Unterseite in Form einer Kufe 12 ausgebildet, mit welcher sie auf der Haut des Patienten aufsitzen. An den einander zugewandten Stirnseiten der Kufen 12 ist eine Leiste 14 angeordnet, die sich über die ganze Breite der Backen 10 erstreckt. Die Leiste 14 ist mittels einer Schwalbenschwanzführung 16 an der Stirnseite der Kufe 12 auswechelbar befestigt. Hierzu kann die Leiste 14 von der Seite in die Schwalbenschwanzführung 16 der Kufe 12 eingeschoben bzw. herausgezogen werden. Die Leiste 14 besteht aus Kunststoff. Vorzugsweise ist eine Rastung vorgesehen, die die Leiste 14 in der eingeschobenen Stellung hält. Hierzu können beispielsweise die Leiste 14 eine kleine Erhebung aufweisen, die in eine Vertiefung der Stirnfläche der Kufe 12 elastisch einrastet.

In die Leiste 14 sind Haken 18 aus Metall eingegossen. Es sind mehrere Haken 18 in einer Reihe nebeneinander in gleichem Abstand über die gesamte Breite der Leiste 14 in diese eingesetzt. Die Haken 18 ragen von der unteren Schmalkante der Leiste 14 nach unten über die untere Gleitfläche der Kufe 12 hinaus und sind mit ihrer scharfen Spitze von der Stirnseite der Leiste 14 weggebogen, so daß die Spitzen der Haken 18 der beiden Backen 10 gegeneinander gerichtet sind. Die Haken 18 werden mit ihren scharfen Spitzen im Bereich der Wundränder in die Cutis eingestochen und verankern die Backen 10 in den Wundrändern.

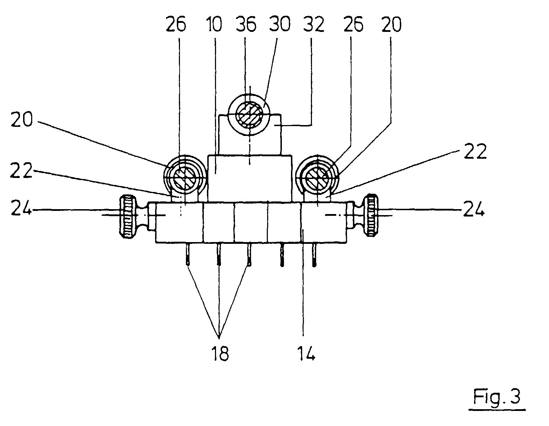

Anstelle einer über die gesamte Breite der Backe 10 durchgehenden Leiste 14 kann diese auch modulartig in einzelne Teile unterteilt sein, die jeweils nur einen Haken 18 oder ganz wenige Haken 18 aufnehmen, wie dies insbesondere in Fig. 3 gezeigt ist. Die Haken 18 können dadurch einzeln in die Wundränder eingestochen werden und die Teile werden nacheinander in die Schwalbenschwanz-Führung 16 eingeführt.

An den beiden Schmalseiten der Backen 10 sind auf deren Oberseite jeweils Führungsbuchsen 20 angeordnet, deren Achse horizontal, d.h. parallel zur unteren Gleitfläche der Kufen 12 verläuft. Die Führungsbuchsen 20 weisen einen senkrecht zu ihrer Achse angeordneten Lagerzapfen 22 auf, der senkrecht zur Ebene der Gleitfläche der Kufen 12 von oben drehbar in einer Bohrung der Backen 10 sitzt. Die Führungsbuchsen 20 sind somit um die Achse des Lagerzapfens 22, d.h. um eine zu der Gleitfläche der Kufe 12 senkrechte Achse drehbar in den Backen 10 gelagert. Rändelschrauben 24, die in die Seitenflächen der Backen 10 eingesetzt sind, greifen in einen Einstich der Lagerzapfen 22, um diese axial in den Backen 10 zu halten und bei Bedarf in ihrer jeweiligen Drehstellung festzuklemmen.

Die zwei Backen 10 sitzen mit jeder ihrer Führungsbuchsen 20 jeweils auf einer Führungsstange 26. Die beiden Backen 10 sind auf diesen Führungsstangen 26 durch die Führungsbuchsen 20 gleitend geführt gegeneinander verschiebbar, wobei die drehbare Lagerung der Führungsbuchsen 20 in den Backen 10 ermöglicht, daß die Backen 10 auch eine von 90° abweichende Schrägstellung gegenüber den Führungsstangen 26 einnehmen können. Die Leisten 14 mit den Haken 18 der beiden Backen 10 können auf diese Weise nicht nur parallel zueinander angeordnet sein, sondern auch in einem Winkel zueinander stehen. Dies ermöglicht die Anpassung der Lage der Haken 18 an den jeweiligen Verlauf der Wundränder.

Die Köpfe 28 von in die Enden der Führungsstange 26 koaxial eingedrehten Schrauben ermöglichen das Einführen der Führungsstangen 26 in die Führungsbuchsen 20 und halten diese unverlierbar auf den Führungsstangen 26.

Mittig auf der Oberseite der Backen 10 ist jeweils eine Gewindebuchse 30 angeordnet, deren Achse ebenfalls horizontal, d.h. parallel zur Gleitebene der Kufe 12 verläuft. Die Gewindebuchsen 30 sitzen mit rechtwinklig zu ihrer Achse angeordneten Lagerzapfen 32 in zur Gleitebene der Kufen 12 senkrecht angeordneten Lagerbohrungen der Oberseite der Backen 10. Die Gewindebuchsen 30 sind somit in der gleichen Weise wie die Führungsbuchsen 20 um eine vertikale, zur Ebene der Gleitfläche der Kufe 12 senkrechte Achse schwenkbar in den Backen 10 gelagert. Eine in die hintere Stirnfläche der Backe 10 eingedrehte Schraube 34 greift in einen Einstich des Lagerzapfens 32, um diesen axial unverlierbar in der Backe 10 zu halten. Die Gewindebuchsen 30 der beiden Backen 10 werden von einer gemeinsamen Gewindespindel 36 durchsetzt, die zu den Führungsstangen 26 parallel verläuft. Die Gewindespindel 36 ist mittig in zwei gegenläufige Gewindeabschnitte unterteilt, so daß die Gewindebuchse 30 der einen Backe 10 mit der Gewindespindel 36 ein Rechtsgewinde bildet, während die Gewindebuchse 30 der anderen Backe 10 mit der Gewindespindel 36 ein Linksgewinde bildet. Bei einer Drehung der Gewindespindel 36 wandern die beiden Backen 10 somit aufeinander zu oder voneinander weg.

Bei der Verwendung des Haut-Distraktors werden zunächst die Lagerzapfen 22 der Führungsbuchsen 20 gelöst, so daß sich die Führungsbuchsen 20 und die Gewindebuchsen 30 auf ihren jeweiligen Lagerzapfen 22 bzw. 32 drehen können. Die Backen 10 werden mit den Haken 18 in den Wundrändern verankert, wobei die Drehbarkeit der Lagerzapfen 22 und 32 eine Ausrichtung der Backen 10 entsprechend dem Verlauf der Wundränder ermöglicht. Sobald die Backen mit den Haken 18 plaziert und verankert sind, werden die Lagerzapfen 22 mittels der Rändelschrauben 24 festgeklemmt, so daß die Backen 10 in ihrer jeweiligen Winkelstellung bezüglich der Führungsstangen 26 festgelegt sind. Nun können die Backen 10 mittels der Gewindespindel 36 auf den Führungsstangen 26 gleitend gegeneinander bewegt werden, um die Wundränder zusammenzuziehen, wobei die Backen 10 mit ihren Haken 18 die dem Verlauf der Wundränder angepaßte Stellung beibehalten.

Nach dem Schließen der Wundränder wird der Distraktor entfernt. Die Leisten 14 mit den Haken 18 werden von den Backen 10 getrennt und entsorgt. Der übrige Distraktor wird gereinigt und sterilisiert. Erst bei einem erneuten Einsatz werden neue Leisten 14 mit Haken 18 in die Backen 10 eingesetzt.

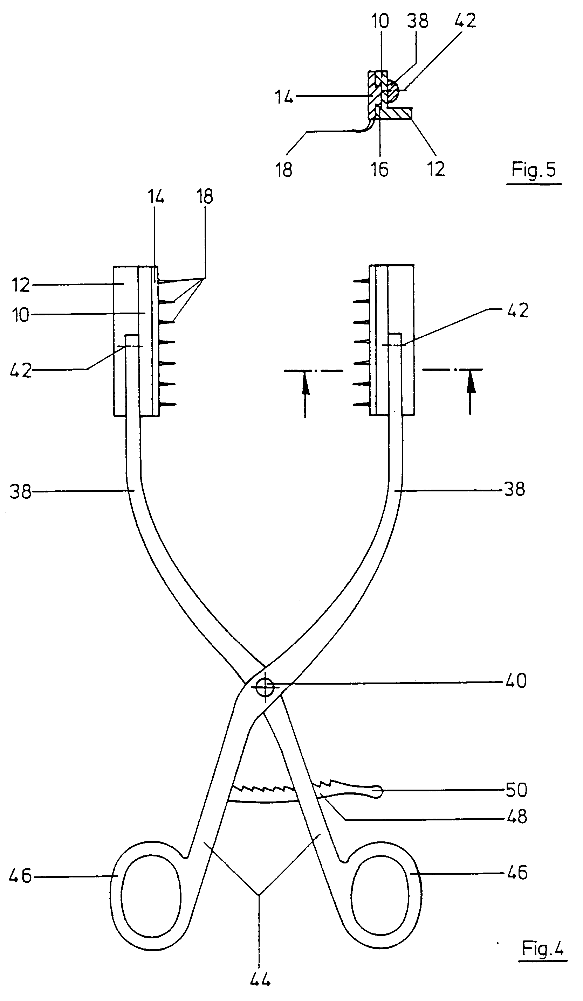

In den Figuren 4 und 5 ist eine weitere Ausführung des Haut-Distraktors dargestellt.

In Übereinstimmung mit dem ersten Ausführungsbeispiel ist an den mit einer Kufe 12 ausgebildeten Backen 10 mittels einer Schwalbenschwanz-Führung 16 eine Leiste 14 mit Haken 18 angebracht. Insoweit wird auf die vorangehende Beschreibung verwiesen.

Jede der beiden Backen 10 ist jeweils an dem freien Ende eines Armes 38 angebracht. Die beiden Arme 38 sind durch ein Gelenk 40 so schwenkbar miteinander verbunden, daß die Backen 10 in der Ebene der Gleitfläche der Kufen 12 gegeneinander verschwenkbar sind.

Die Arme 38 sind an den Backen 10 jeweils mittels eines Gelenkes 42 so angelenkt, daß sie um eine in der Bewegungsrichtung der Backen 10 verlaufende Achse gegenüber den Backen 10 verschwenkbar sind. Im Ausführungsbeispiel der Figur 4 sind die Arme 38 über das Gelenk 40 hinaus zu einem Scherengriff 44 mit Fingerösen 46 verlängert. Der Scherengriff 44 ist mit einer Rastung ausgebildet, wozu an einer Branche des Scherengriffes 44 eine Zahnstange angebracht ist, die federnd in einen Rastzahn der anderen Branche eingreift und mittels eines Griffes 50 aus der Rastung herausgehoben werden kann.

Der Distraktor in dieser Ausführung wird in der in Figur 4 gezeigten Stellung an den Wundrändern angesetzt, wozu die Leisten 14 mit ihren Haken 18 in der Haut verankert werden. Mittels des Scherengriffes 44 werden die Backen 10 gegeneinander bewegt, um die Wundränder zusammenzuziehen. Das Zusammenziehen kann dabei schrittweise in der Teilung der Zahnstange 48 erfolgen. Die Rastung der Zahnstange 48 hält dabei die Arme 38 mit den Backen 10 gegen die Zugspannung der Haut.

Wenn die Wundränder genügend zusammengezogen sind und sich berühren, können die Wundränder miteinander vernäht werden. Hierzu kann im Bedarfsfalle der Scherengriff 44 mit den Armen 38 aus der in Figur 4 gezeigten zur Gleitfläche der Kufen 12 parallelen Ebene hochgeschwenkt werden in eine Stellung, in welcher die Arme 38 und der Scherengriff 44 senkrecht zur Gleitebene der Kufen 12 stehen. Der Scherengriff 44 mit den Armen 38 ist in dieser Stellung nicht mehr störend im Wege, so daß die Wundränder beiderseits der Backen 10 zugänglich sind und von beiden Seiten bis an die Backen 10 hin vernäht werden können.

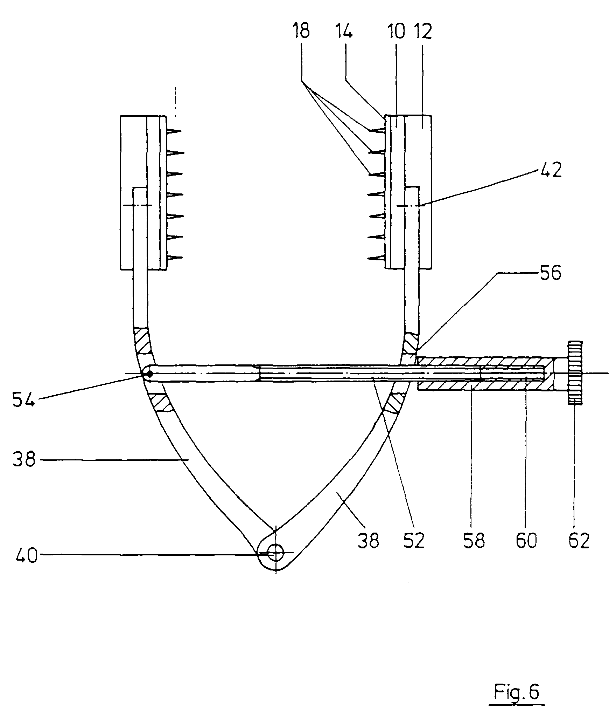

In Figur 6 ist eine weitere Ausführung des Haut-Distraktors gezeigt. Diese stimmt in wesentlichen Teilen mit dem Ausführungsbeispiel der Figur 4 überein, so daß insoweit auf die Beschreibung der Figur 4 und 5 verwiesen wird.

Im Gegensatz zum Ausführungsbeispiel der Figur 4 greifen bei dem Ausführungsbeispiel der Figur 6 die Stellmittel zum Verstellen des Abstandes der Backen 10 an den Armen 38 an. Hierzu ist in dem einen Arm 38 eine Gewindestange 52 mit ihrem einen Ende um einen Gelenkpunkt 54 schwenkbar gelagert. Die Gewindestange 52 ist dabei in der Ebene der Schwenkbewegung der Arme 38 schwenkbar. Die Gewindestange 52 durchsetzt frei ein Langloch 56 des anderen Armes 38, so daß die Gewindestange 52 die gegenseitige Schwenkbewegung der Arme 38 nicht behindert. Auf das durch das Langloch 56 hindurchragende freie Ende der Gewindestange 52 ist ein Stellbolzen 58 mit einer koaxialen Gewindebohrung 60 aufgeschraubt. Der Stellbolzen 58 stützt sich mit seinem auf die Gewindestange 52 aufgeschraubten Ende außen an dem Arm 38 ab. Der Stellbolzen 58 kann mittels eines Rändelknopfes 62 verdreht und damit auf der Gewindestange 52 verschoben werden.

Der Haut-Distraktor der Figur 6 wird in gleicher Weise, wie dies bei Figur 4 beschrieben ist, in die Wundränder eingesetzt. Durch Verdrehen des Stellbolzens 58 werden die Arme 38 gegeneinander verschwenkt und die in den Wundrändern verankerten Backen 10 aufeinander zubewegt. Der Stellbolzen 58 stützt dabei die Arme 38 gegen die Spannung der Haut der Wundränder ab.

Soll die Wunde mittels des Haut-Distraktors durch Gewebsneubildung an den Wundrändern verschlossen werden, so werden die Backen des Distraktors nur in solchen Schritten und in solchem zeitlichem Abstand gegeneinander bewegt, daß die Gewebsneubildung dieser Bewegung der Wundränder folgen kann. Die Verstellung der Backen kann dabei manuell durch Verstellen des Gewindes (in den Ausführungsbeispielen der Figur 1 bis 3 und Figur 6) bzw. durch Schließen des Scherengriffes (im Ausführungsbeispiel der Figur 4) nach empirischen Werten erfolgen. Um das Verfahren zuverlässiger und weniger von der Erfahrung abhängig zu machen, kann die Gewebsneubildung mittels eines geeigneten Sensors überwacht werden. Ein solcher Sensor kann beispielsweise die Durchblutung des Gewebes oder die Sauerstoffversorgung bzw. den Sauerstoffpartialdruck in dem Gewebe in dem Bereich ermitteln, der durch den Haut-Distraktor gedehnt wird. Die Verstellung der Backen erfolgt dann in solchen Schritten, daß die für eine Überdehnung der Haut charakteristischen Werte nicht überschritten werden.

Auch hier kann das Nachstellen des Distraktors manuell entsprechend den über den Sensor ermittelten Werten erfolgen.

Die Überwachung der Hautdehnung ermöglicht auch ein automatisches Nachstellen des Haut-Ditraktors, sofern ein motorischer Antrieb des Distraktors vorgesehen ist. Für einen solchen motorischen Antrieb eignen sich insbesondere die Ausführungen mit Gewinde-Stellmitteln, z.B. gemäß den Figuren 1 bis 3 und Figur 6.

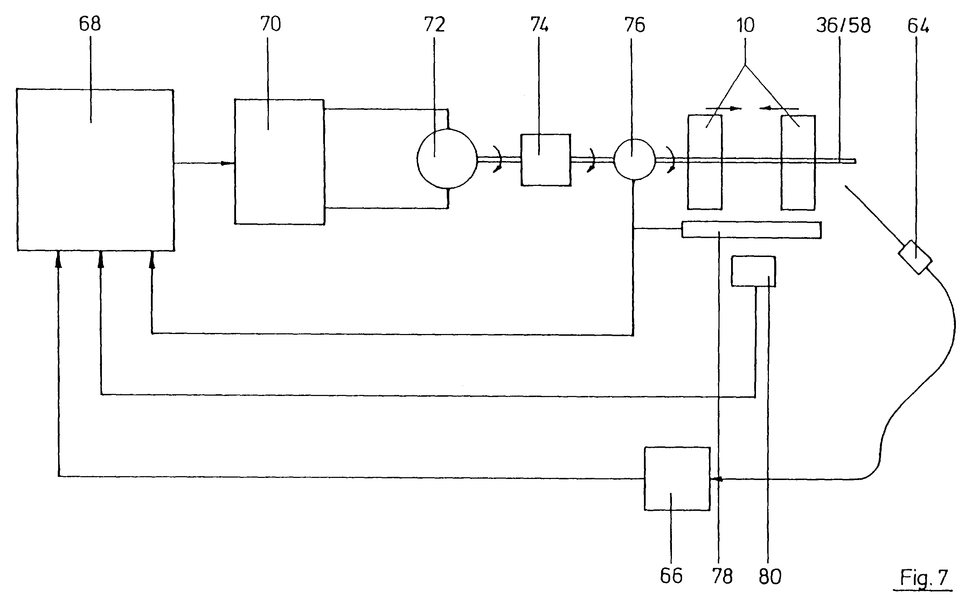

In Figur 7 ist schematisch eine Anordnung zur automatischen Verstellung des Haut-Distraktors in einer solchen Ausführung dargestellt.

Die Backen 10 des Distraktors werden über Gewinde-Stellmittel, z.B. eine Gewindespindel 36 gemäß der Ausführung der Figuren 1 bis 3 oder einen Stellbolzen 58 gemäß der Ausführung der Figur 6 verstellt. Ein Sensor 64 mißt den Zustand des Hautgewebes in dem gedehnten Bereich, d.h. auf der von der Wunde abgewandten Seite der Backen 10. Der Sensor 64 mißt beispielsweise den Sauerstoffpartialdruck im Gewebe oder die Durchblutung des Gewebes über optische Mittel. Die Meßwerte des Sensors 64 werden in einem Meßwertewandler 66 in digitale Werte umgewandelt, die einem Rechner 68 zugeführt werden. Der Rechner 68 steuert über eine Steuerung 70, einen Motor 72, der über ein Getriebe 74 die Gewinde-Stellmittel 36/58 des Distraktors betätigt. Die Rückmeldung der Verstellung der Backen 10 zu dem Rechner 68 erfolgt über einen an die Gewinde-Stellmittel angekoppelten Encoder 76 oder ein den Abstand der Backen 10 bestimmendes Weglängenmeßsystem 78. Gegebenenfalls kann auch zusätzlich zu der Messung der Gewebe-Beschaffenheit mittels des Sensors 64 oder anstatt dieser Messung eine Kraftmeßeinrichtung 80 vorgesehen sein, die die auf die Backen 10 einwirkende Zugkraft der gedehnten Haut bestimmt und dem Rechner 68 als Ist-Wert zuführt.

## Patentansprüche

1. Haut-Distraktor, mit wenigstens einem Paar von Backen (10), mit Stellmitteln (30, 36; 52, 58) zum Verstellen des gegenseitigen Abstandes der Backen (10), mit an den Backen (10) auswechselbar angeordneten Teilen (14) und mit quer zur Verstellrichtung der Backen (10) nebeneinander an den Teilen (14) angebrachten in die Haut einstechbaren Haken (18), dadurch gekennzeichnet, daß mehrere Teile (14), an denen jeweils nur ein, zwei oder einige wenige Haken (18) angebracht sind, nacheinander modulartig aneinander anschließend in einer quer zur Verstellrichtung verlaufenden Führung (16) der Backen (10) befestigbar sind, um eine Reihe der Haken (18) zu bilden.

2. Haut-Distraktor nach Anspruch 1, dadurch gekennzeichnet, daß die Führung eine Schwalbenschwanz-Führung (16) ist.

3. Haut-Distraktor nach Anspruch 1 oder 2, dadurch gekennzeichnet, daß die Teile (14) aus Kunststoff bestehen, und die Haken (18) in diese Teile (14) eingegossen sind.

## Claims

1. A skin stretching device, having at least one pair of jaws (10), having adjustment means (30, 36; 52, 58) for adjusting the mutual spacing of the jaws (10), having parts (14) disposed in an exchangeable manner on the jaws (10) and having hooks (18), which are mounted next to one another on the parts (14) and transversely to the adjustment direction of the jaws (10) and can be inserted into the skin,
**characterised in that** several parts (14), on each of which only one, two or a few hooks (18) are mounted, can be attached one after another, modular-fashion adjacent to one another in a guide (16) of the jaws (10) which extends transversely to the adjustment direction, in order to form a row of the hooks (18).

2. A skin stretching device according to Claim 1,
**characterised in that** the guide is a dovetail guide (16).

3. A skin stretching device according to Claim 1 or 2,
**characterised in that** the parts (14) are made from plastics, and the hooks (18) are cast into these parts (14).

## Revendications

1. Dispositif permettant de resserrer la peau, comportant au moins une paire de mâchoires (10), avec des moyens de réglage (30, 36; 52, 58) pour assurer le réglage de l'espacement mutuel des mâchoires (10), avec des parties (14) disposées de façon remplaçables sur les mâchoires (10) et avec des crochets (18) susceptibles d'être enfichées dans la peau, montés les uns à côté des autres sur les parties (14), transversalement par rapport à la direction de réglage des mâchoires (10),
caractérisé en ce que
plusieurs parties (14) sur chacune desquelles n'est monté qu'un, deux, ou quelques crochets (18) en petit nombre sont susceptibles d'être fixées les uns après les autres, en modules, en se suivant les uns aux autres, dans un guidage (16), s'étendant transversalement par rapport à la direction de réglage des mâchoires (10) pour constituer une rangée de crochets (18).

2. Dispositif permettant de resserrer la peau selon la revendication 1,
caractérisé en ce que
le guidage est un guidage à queue d'aronde (16).

3. Dispositif permettant de resserrer la peau selon la revendication 1 ou la revendication 2,
caractérisé en ce que
les parties (14) sont réalisées en matière plastique et les crochets (18) sont insérés dans ces parties (14) lors du moulage.
